# EUROPÄISCHE PATENTSCHRIFT

(11) **EP 2 529 425 B1**
(45) Veröffentlichungstag und Bekanntmachung des Hinweises auf die Patenterteilung: **31.08.2016**
(21) Anmeldenummer: 10792859.0
(22) Anmeldetag: 19.11.2010
(51) Int. Cl.: H01L 51/54, C07F 15/00

(54) **VERWENDUNG DES GUANIDINIUM-KATIONS IN EINEM LICHTEMITTIERENDEN BAUELEMENT**
USE OF A GUANIDINIUM CATION IN A LIGHT-EMITTING COMPONENT
UTILISATION DU CATION GUANIDINIUM DANS UN ÉLÉMENT STRUCTURAL ÉLECTROLUMINESCENT

(30) Priorität: 25.01.2010 DE 102010005634
(43) Veröffentlichungstag der Anmeldung: 05.12.2012
(73) Patentinhaber: OSRAM GmbH, 80807 München (DE)
(72) Erfinder: SCHMID, Günter, 91334 Hemhofen (DE); HARTMANN, David, 91056 Erlangen (DE); KANITZ, Andreas, 91315 Höchstadt (DE); SARFERT, Wiebke, 91074 Herzogenaurach (DE)
(74) Vertreter: Epping - Hermann - Fischer
(86) Internationale Anmeldenummer: PCT/EP2010/067829
(87) Internationale Veröffentlichungsnummer: WO 2011/088918

(56) Entgegenhaltungen:
- WO-A1-2010/007107
- WO-A2-2008/141637
- DE-A1-102013 202 250
- US-A1- 2002 099 157
- US-A1- 2006 054 886
- US-A1- 2007 176 148
- OUISSE T ET AL: "Double-layer formation in organic light-emitting electrochemical cells", JOURNAL OF APPLIED PHYSICS, AMERICAN INSTITUTE OF PHYSICS. NEW YORK, US, Bd. 92, Nr. 5, 1. September 2002 (2002-09-01), Seiten 2795-2802, XP012057194, ISSN: 0021-8979, DOI: DOI:10.1063/1.1499201

## Beschreibung

Die Erfindung betrifft eine Anwendung des Guanidinium-Kations und ein lichtemittierendes Bauelement.

Lichtemittierende Bauelemente auf organischer Basis sind bekannt, jedoch besteht der Bedarf diese hinsichtlich Stabilität und Lebensdauer einerseits und hinsichtlich Kosten andererseits zu optimieren.

Die Druckschrift D6 (WO 2010/007107 A1) beschreibt phosphoreszente Metallkomplexverbindungen, Herstellungsverfahren hierfür und ein entsprechendes strahlungsmeittierendes Bauelement.

Die Druckschrift D2 (Ouisse T. et al.: Journal of Applied Physics; Vol. 92; No. 5; September 1, 2002; pp. 2795-2802) beschreibt OLEECs mit Guanidinium Salz in einer aktiven Schicht.

Die Druckschrift D3 (US 2002/0099157 A1) betrifft Monomere, Polymere umfassend solche Monomere und deren Anwendung in OLEECs.

Die Druckschrift D4 (US 2007/0176148 A1) betrifft OLEDs mit Guanidinium Salzen in einer emittierenden Schicht.

Neben der Bedeutung des Guanidinium-Kations als Dehydrant von Proteinen und in der Biochemie gibt es Anwendungen des Guanidinium-Kations in Batterien und elektrochemischen Zellen. Diese Anwendung von Guanidinium- Salzen ist beispielsweise in EP1363345A2 und US20030211389A1 beschrieben. Hierbei dienen die Guanidinium- Salze zur Herstellung von Elektrolyten für die elektrochemische Speicherung von Ladung in Akkumulatoren und Batterien.

Bei dieser Anwendung steht die Eigenleitfähigkeit des Leitsalzes im Mittelpunkt, sie soll möglichst hoch sein, um die Ladung effektiv speichern zu können.

Es gibt jedoch sehr viele Guanidinium-Salze mit geringer Eigenleitfähigkeit, für die momentan keine andere Anwendung als die als Synthesebaustein und zur Dehydrierung bei Proteinen bekannt ist.

Aufgabe der vorliegenden Erfindung ist es deshalb, eine Anwendung des Guanidinium-Kations zu finden, bei der auch Guanidinium -Salze mit geringer Eigenleitfähigkeit hohe Effizienz zeigen.

Lösung der Aufgabe und Gegenstand der Erfindung ist die Anwendung der Guanidinium-Salze in einem lichtemittierenden organischen elektronischen Bauelement. Außerdem ist Gegenstand der Erfindung eine organische elektrochemische Zelle die in ihrer aktiven organischen Schicht eine, ein Guanidinium-Kation enthaltende, Verbindung aufweist.

Das zentrale Stickstoffatom des Guanidiniumgrundgerüstes trägt immer eine positive Ladung, die je nach Ausführung, über die gesamte Verbindung delokalisiert sein kann, oder am zentralen Kohlenstoff umgeben mit den drei Guanidiniumstickstoffatomen lokalisiert ist. Im letzten Fall ist die Ladung nur über 4 Atome (1x N und 3 x C) delokalisiert.

Emitter und Materixmaterialien gemäß dieser Erfindung enthalten als zentrale Einheit mindestens eine Guanidiumeinheit mit positiver Ladung wie unten gezeigt. Die positive Ladung ist dabei naturgemäß mindestens innerhalb der Guanidiniumeinheit delokalisiert.

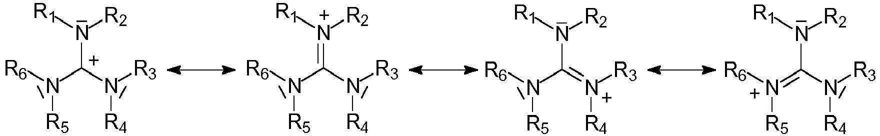

Dies ist die zentrale Guanidinium-Einheit der erfindungsgemäßen Matrix- und Emittermaterialien wobei alle Reste

R = unabhängig voneinander- H, gesättigte oder ungesättigte verzweigte Alkylreste, unverzweigte Alkylreste, kondensierte Alkylreste, ringförmige Alkylreste, vollständig oder teilweise substituierte unverzweigte, verzweigte, kondensierte und/oder ringförmige Alkylreste, Alkoxygruppen, Amine, Amide, Ester, Carbonate, Aromaten, vollständig oder teilweise substituierte Aromaten, Heteroaromaten, kondensierte Aromaten, vollständig oder teilweise substituierte kondensierte Aromaten, Heterocyclen, vollständig oder teilweise substituierte Heterocyclen, kondensierte Heterocyclen, Halogene, Pseudohalogene.

Alle Substituenten R₁, R₂, R+ können unabhängig von einander ausgewählt sein aus den oben genannten Resten, es handelt sich bevorzugt um C₁ bis C₂₀, kondensierte z.B. decahydronaphtyl-, adamantyl-, cyclischer, cyclohexyl-, oder voll oder teilweise substituierter Alkylrest, bevorzugt C₁ bis C₂₀. Diese Ketten oder Gruppen können verschiedene Endgruppen tragen, beispielsweise geladene Endgruppen wie SOₓ^{-,} NR⁺ und so weiter.

Die Alkylreste können wiederum Gruppen wie Ether, Ethoxy-, Methoxy-, etc. Ester-, Amid-, Carbonat-, etc. oder Halogene, bevorzugt Fluor, tragen. R₁, R₂ und R₃ sollen aber nicht auf Alkylreste beschränkt sein, sondern kann ebenso gut substituierte oder unsubstituierte aromatische Systeme umfassen, wie beispielsweise Phenyl-, Biphenyl-, Naphtyl-, Phenanthryl-, Benzyl-, und so weiter.

Die Substituenten R₁ bis R₆ können unabhängig voneinander aliphatisch, aromatisch oder komplex aufgebaut sein.

Durch Variation der der Reste R₁, bis R₆ lassen sich verschiedenartige Ligandsysteme erzeugen, die geeignet für die Emittersysteme im erfindungsgemäßen Bauteil sind. R₁, bis R₆ können dabei unabhängig voneinander H (eingeschränkt, da normalerweise Guanidiniumsalze vollständig durchsubstituiert sind - Methyl-, Ethyl- verallgemeinert unverzweigte, verzweigte, kondensierte (Decahydronaphthyl-), ringförmige (Cyclohexyl-) oder ganz oder teilweise substituierte Alkylreste (C₁ - C₂₀) sein. Diese Alkylreste können Ethergruppen (Ethoxy-, Methoxy-, usw.), Ester-, Amid-, Carbonatgruppen etc. oder
auch Halogene, insbesondere F enthalten. Im Sinne der Erfindung sind auch substituierte oder unsubstituierte aliphatische Ringe bzw. Ringsysteme, wie Cyclohexyl.

R₁ bis R₆ sind nicht auf gesättigte Systeme beschränkt, sondern beinhalten auch substituierte bzw. unsubstituierte Aromaten wie Phenyl, Diphenyl, Naphthyl, Phenanthryl etc. bzw. Benzyl etc. Eine Zusammenstellung als Substituenten in Frage kommender Heterocyclen ist in Tabelle 1 dargestellt. Der Einfachheit halber ist nur der Grundkörper der Aromaten dargestellt. Prinzipiell kann dieser Grundkörper mit weiteren Resten R substituiert sein, die sich analog von den hier definierten Reste R₁ bis R₆ ableiten lassen.

Die Erfindung betrifft die Verwendung eines Salzes, das ein Guanidinium-Kation enthält als phosphoreszentes Emittersystem in einem lichtemittierenden organischen elektronischen Bauelement ist das lichtemittierende organische elektronische Bauelement eine organische elektrochemische Zelle ist, wobei das, das Guanidinium-Kation enthaltende Salz die folgende Struktureinheit zeigt: 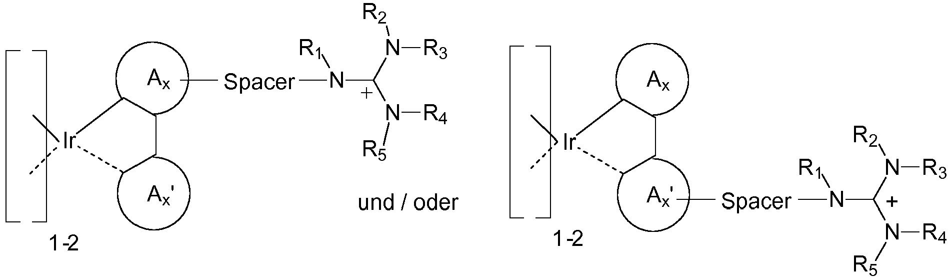 wobei
A = ein substituierter oder unsubstituierter Aromat oder Heteroaromat, der zu den genannten Bindungsverhältnissen befähigt ist,
als Spacer eingesetzt werden:
   a) ungesättigte Alkylketten mit 1 - 20 Kohlenstoffatomen und konjugierten Doppelbindungen,
   b) ungesättigte Alkylketten mit 1 - 20 Kohlenstoffatomen und konjugierten Dreifachbindungen, oder
   g) gemischte Varianten aus a und b,
wobei die Substituenten R₁ bis R₅ unabhängig voneinander aliphatisch, aromatisch oder komplex aufgebaut sein können und insbesondere
   R = unabhängig voneinander- H, gesättigte oder ungesättigte, verzweigte Alkylreste, unverzweigte Alkylreste, kondensierte Alkylreste, ringförmige Alkylreste, vollständig oder teilweise substituierte unverzweigte, verzweigte, kondensierte und/oder ringförmige Alkylreste, Alkoxygruppen, Amine, Amide, Ester, Carbonate, Aromaten, vollständig oder teilweise substituierte Aromaten, Heteroaromaten, kondensierte Aromaten, vollständig oder teilweise substituierte kondensierte Aromaten, Heterocyclen, vollständig oder teilweise substituierte Heterocyclen, kondensierte Heterocyclen, Halogene, Pseudohalogene sein kann.

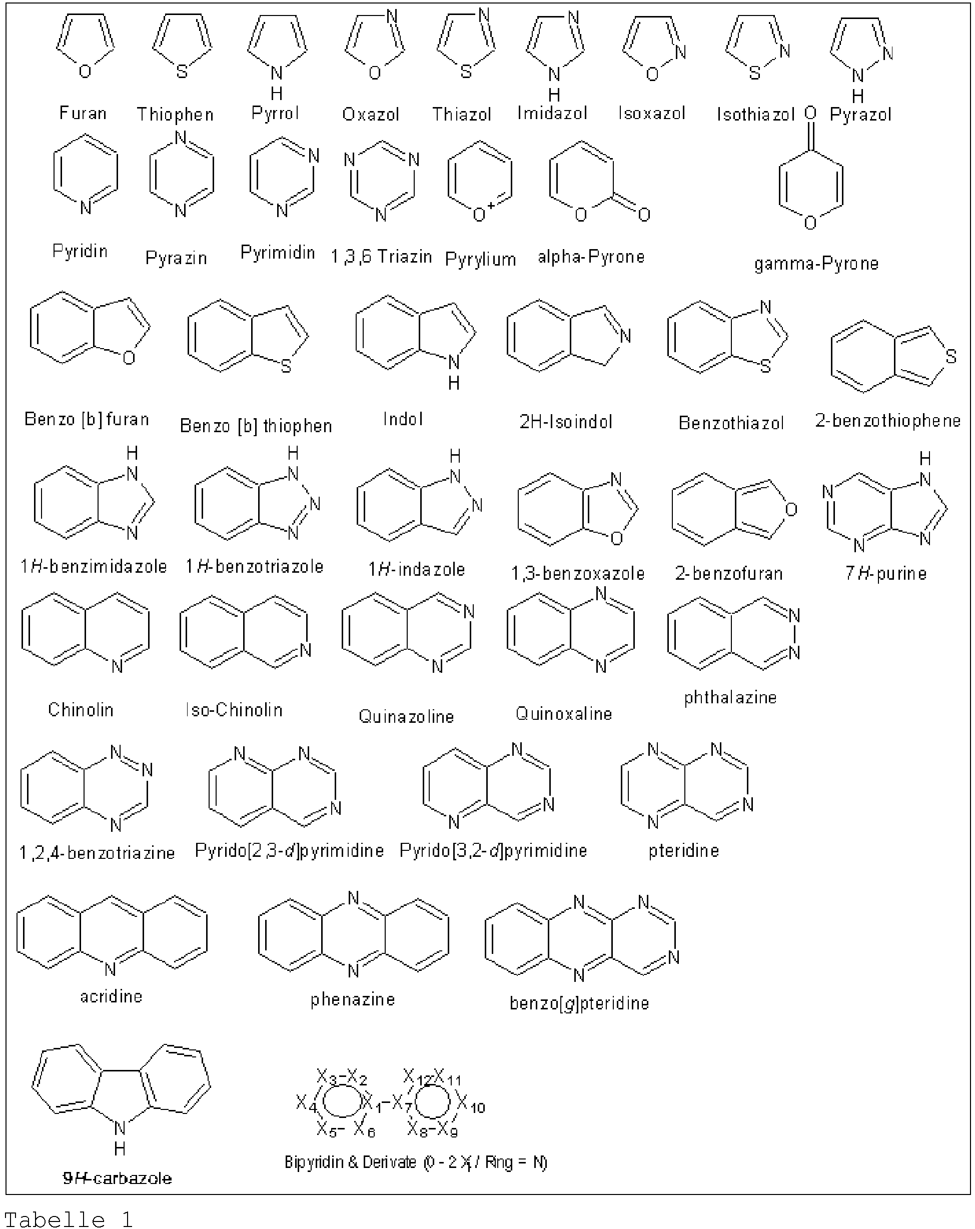

Tabelle 1 zeigt eine Auswahl substituierter bzw. unsubstituierter Heterozyklen, die als Reste R₁, R₂ unabhängig voneinander in Frage kommen. Der Einfachheit halber ist nur die Grundeinheit dargestellt. Die Bindung an den Liganden kann an jeder bindungsfähigen Stelle des Grundkörpers erfolgen.

R+ führt die positive Ladung am Stickstoffatom ein und ist bevorzugt ein substituierter oder unsubstituierter aliphatischer Rest wie beispielsweise ein Methyl-, Ethyl- oder ein allgemeiner geradkettiger oder verzweigter, kondensierter (decahydronaphthyl-, adamantyl-), ring-förmiger (cyclohexyl-) Alkylrest, der vollständig oder teilweise substituiert sein kann und Alkyl-Einheiten mit beispielsweise 1 bis 20 Kohlenstoffatome umfasst.

Bevorzugt aber nicht einschränkend werden die zur Kompensation der positiven Ladung benötigten Anionen ausgewählt aus: Fluorid, Chlorid, Bromid, Jodid, Sulfat, Phosphat, Carbonat, Trifluormethansulfonat, Trifluoracetat, Tosylat, Bis(trifluormethylsulfon)imid, Tetraphenylborat, B₉C₂H₁₁²; Hexafluorophosphat, Tetrafluoroborat, Hexafluoroantimonat, Tetrapyrazolatoborat.

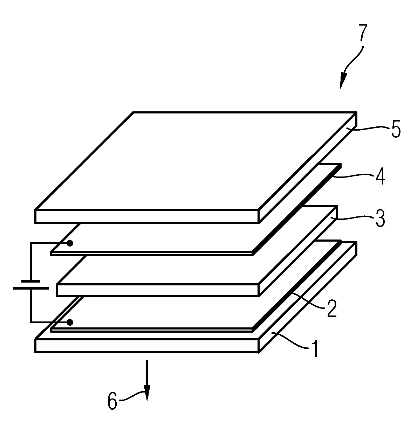

Die Guanidniumderivaten können selbst aus kondensierten Ringsystemen bestehen oder enthalten. Ein vollständig anneliertes System ist in Fig.4 dargestellt.

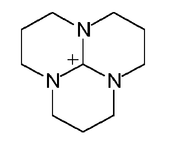

Hier ist ein vollständig anneliertes System mit Guanidiniumzentraleinheit gezeigt. Der Einfachheit halber wurden alle möglichen Substituenten weggelassen. Prinzipiell kann jede C-H-Bindung durch eine C-R Bindung ersetzt werden.

Aus den gezeigten Strukturen lassen sich eine ganze Reihe von Systemen ableiten, die alle den Guanidiniumgrundkörper enthalten. Unten gezeigt sind beispielhaft annelierte Systeme, die vom Imidazol abgeleitet wurden.

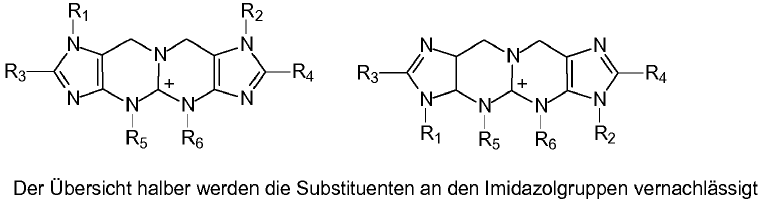

Oben sieht man annelierte Guanidiniumkationen mit ankondensierten Imidazolringen bzw. teilweise hydrierter Imidazolen.

Die unten stehenden Strukturen zeigen eine Auswahl der Vielfalt ableitbarer Strukturen im Sinne der Erfindung.

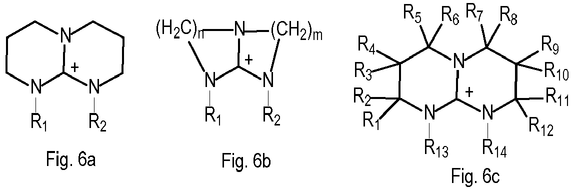

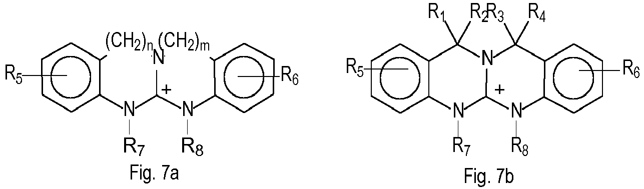

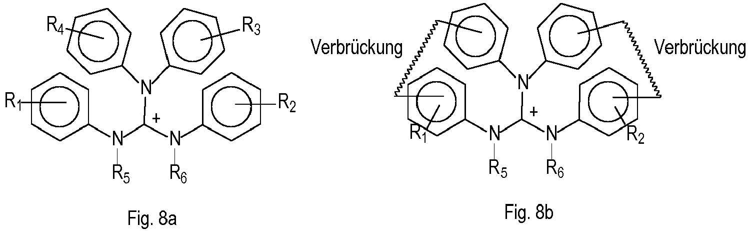

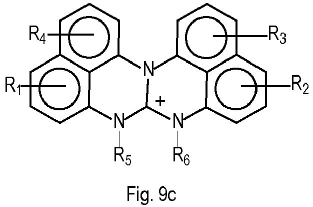

Die bisher diskutierten Verbindungen dienen vornehmlich als Matrixmaterialien, da sie keine emissiven Einheiten enthalten.

Dabei ist durchaus im Sinne der Erfindung denkbar, dass die aromatischen Substituenten (konjugiert bzw. nicht konjugiert zum Guanidiniumkern sein) eine entsprechende Größe besitzen können, bzw. ein Donor-Akzeptorpaar bilden können, das eine fluoreszente Einheit bilden kann, die analog zur OLED auch elektrisch anregbar ist.

Die aktive organische Schicht einer OLEEC umfasst eine Matrix und ein darin gebundenes (physikalisch oder chemisch) Emittermaterial. Gemäß der Erfindung kann ein Guanidinium-Kation sowohl in dem Matrixmaterial als auch in dem Emittermaterial, so wie auch in beiden, vorliegen.

Der typische Aufbau einer organischen elektrochemischen Zelle OLEEC umfasst ein transparentes Substrat, darauf eine transparente Elektrode, die meist als Anode eingesetzt wird, darauf die aktive organische Schicht, die beispielsweise eine Schichtdicke im Bereich von 100nm bis 1µm hat. Auf der aktiven organischen Schicht liegt die zweite, nicht zwangsläufig transparente Elektrode, die meist als Kathode geschaltet wird.

Im Gegensatz zu den weithin bekannten und bereits vielfach diskutierten OLEDs, die Elektronen/Loch-transportierende Schichten und Elektronen/Lochblockierende Schichten neben den emittierenden Schichten umfassen, zeichnen sich die OLEECs vor allem durch einen wesentlich einfacheren Aufbau aus, da hier meist nur eine organische aktive Schicht benötigt wird.

In den organischen Licht emittierenden Dioden (OLEDs) wird, insbesondere bei den mit so genannten small molecules aufgebauten OLEDs, ein so genannter Multilayer-Aufbau realisiert, weil zusätzlich zu der Licht emittierenden Schicht auch noch effizienzerhöhende Schichten wie Loch- und/oder Elektroneninjektionsschichten bzw. Blockerschichten zwischen den Elektroden zum besseren Übergang der Ladungsträger angeordnet werden. Oftmals werden dabei hochreaktive Materialien eingesetzt, weil die Materialien sich durch niedrige Austrittsarbeit auszeichnen. Deshalb ist eine hermetische Verkapselung bei OLEDs essentiell.

Da bei den OLEECs auf die reaktiven Elektroden der OLED verzichtet werden kann, ist die gesamte Verkapselungsproblematik bei den OLEECs nicht so schwierig wie bei den OLEDs. Die OLEECs gelten daher als zukunftsträchtiger Ersatz für die OLEDs.

In OLEDs werden neutrale organische, anorganische und/oder metallorganische Verbindungen als Matrix, Emitter und Dotierstoffe genutzt. Im Gegensatz ist die Materialbasis organischer lichtemittierender ektrochemischer Zellen ionischer Natur. Zum Aufbau des Bauelements werden die ionischen Emittersysteme entweder in einer neutralen (z.B. neutrale Kunststoffe wie Polymethylmethacrlat) oder ionischen (z.B. ionische Flüssigkeiten) Matrix eingebracht und mittels zweier Elektroden kontaktiert.

Ganz generell haben organische elektrolumineszente Elemente zumindest eine organische Schicht, die sich zwischen zwei Elektroden befindet. Sobald Spannung an die Elektroden angelegt wird, werden Elektronen von der Kathode in die untersten unbesetzten Molekülorbitale der organischen Licht emittierenden Schicht injiziert und wandern auf die Anode zu.

Korrespondierend dazu werden Löcher von der Anode in die obersten besetzten Molekülorbitale der organischen Schicht injiziert und wandern entsprechend zur Kathode. In den Fällen, wo sich wanderndes Loch und wanderndes Elektron innerhalb der organischen Licht emittierenden Schicht auf einem lichtemittierenden Stoff treffen, entsteht ein Exciton, das unter Lichtemission zerfällt. Damit das Licht überhaupt aus dem elektrolumineszierenden Element austreten kann, muss zumindest eine Elektrode transparent sein, in den meisten Fällen ist das eine Elektrode aus Indium-Zinn-Oxid, die als Anode eingesetzt wird. Die ITO-Schicht wird normalerweise auf einem Glasträger abgeschieden.

Die Degradationsmechanismen in OLEECs und die Entwicklung stabiler Matrix- und Emittersysteme für OLEECs ist derzeit Gegenstand zahlreicher Untersuchungen. Emitter- und Matrixsysteme mit Guanidinium-Einheiten lassen aufgrund deren Stabilität eine Erhöhung der Lebensdauer von OLEECs erwarten.

Bei der Anwendung der aus einem Guanidinium-Kation gebildeten Salze für elektrochemische Zellen sollte die Eigenleitfähigkeit relativ gering sein, da sonst ein zu hoher Leckstrom durch das Bauteil fließen würde. Die in dieser Erfindungsmeldung vorgestellten Guanidiniumverbindungen besitzen meist geringe Eigenleitfähigkeiten, ermöglichen aber den Ladungstransport durch das Bauelement durch "Hopping". Insbesondere ermöglichen Sie den Ladungstransport über die Matrix zum Emitter.

Nach einer vorteilhaften Ausführungsform der Erfindung enthält der Emitter unabhängig von der Matrix auch eine Guanidinium- Einheit.

Die OLEECs, wie bereits erwähnt, kommen grundsätzlich mit nur einer aktiven organischen Schicht aus, die noch dazu über massenfertigungstaugliche Methoden wie einfache Beschichtungsmethoden, beispielsweise spin-coating, Rakeln, Eintauchen, "slot die coating" oder Druckmethoden wie Siebdrucken, Flexographie, Tiefdruck, Tintenstrahldrucken aufbringbar ist.

Die typische Schichtdicke ist 10 bis 200 nm, kann aber auch andere Werte beispielsweise bis zu 1000 nm annehmen. Da in OLEECs das elektrische Feld vornehmlich über die Elektroden und nicht über die lichtemittierende Schicht abfällt, können auch Schichtdicken über 1000nm noch gute Bauelemente liefern. Vorzugsweise müssen für OLEECs nicht die reaktiven Elektroden wie Barium, Calcium, Lithiumfluorid, Cäsiumfluorid, Magnesium etc. eingesetzt werden, sondern es können luftstabile Metalle wie Gold, Palladium, Platin Aluminium, Magnesium, Silber, Kupfer, Nickel, Eisen, ITO, AZO, und deren Legierungen eingesetzt werden.

Bei den OLEECs ist die aktive Schicht ein Gemisch aus einem Leiter für Ionen und Elektronen und einer emittierenden Spezies. Ionische Übergangsmetallkomplexe (iTMC) vereinigen diese Anforderungen und werden daher oft in OLEEC-Anwendungen eingesetzt. Ein typischer Vertreter ist das von Q. Pei, G. Yu, C. Zhang, Y. Yang, A.J. Heeger in Science, Vol. 269, 1086-1088, 1995 beschriebene Ruthenium Tris-Bipyrdidin Hexafluorophosphat [Ru(bpy)₃]²⁺(PF₆⁻)₂.

Wenn ein elektrisches Feld an iTMCs angelegt wird, dann gruppieren sich die Ionen im elektrischen Feld um. Als Ergebnis wird ein hohes elektrisches Feld an den Elektroden gebildet, so dass beide Kontakte ohmsche Kontakte werden, wodurch der Ladungsübergang in die organische Schicht erleichtert wird. Die Ladungen werden dann über "hopping" durch die iTMC Schicht transportiert. Wenn Löcher und Elektronen aufeinandertreffen, rekombinieren sie unter Bildung von Excitonen, wodurch Lichtemission entsteht.

Die meisten iTMC-Materialien die bislang bekannt sind, haben Ruthenium, Osmium, Kupfer, oder Iridium Zentralatome.

Im Folgenden werden die klassischen OLED Komplexe mit der gezeigten Formel I abgekürzt. Stellvertretend für alle emittierenden Systeme werden Iridiumkomplexe mit der Koordinationszahl 6 abgebildet. Unter Berücksichtung der Koordinationszahl z.B. Platin 4, Europium meist 8, Os 6, gelten die Betrachtungen auch für andere Systeme. Die Kohlenstoff-6-Bindung wird dabei als durchgezogene Linie, die koordinative Bindung des zweizähnigen Liganden gestrichelt dargestellt. Die Formel zeigt diese Verhältnisse stark schematisiert. Mit "A" wird ein substituierter oder unsubstituierter Aromat oder Heteroaromat bezeichnet, der zu den genannten Bindungsverhältnissen befähigt ist. Alle Aromaten können wie im Stand der Technik gezeigt, unabhängig voneinander variiert werden. Die durchgezogene Bindung zu Kohlenstoff oder Stickstoff wird aus einer C-H oder N-H Vorstufe durch Wasserstoffabspaltung (Zyklometallierung) gebildet. Die gestrichelte Linie deutet entweder einen Carbenkohlenstoff oder einen durch ein freies Elektronenpaar koordinierenden Stickstoff oder Phosphor an.

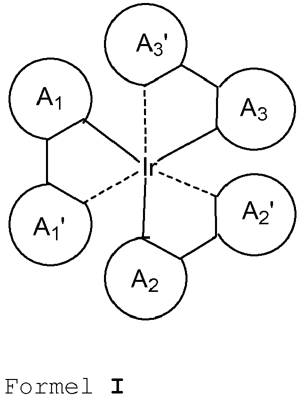

Gemäß der Erfindung sind die unten gezeigten Emittereinheiten beispielsweise über eine Guanidiniumeinheit verbunden und/oder haben als Rest Rₓ einen Guanidiniumrest. Damit, wie auch möglicherweise durch R+ am Stickstoff erhalten die in der Regel neutralen Emitter eine Ladung.

Die klassischen Iridiumemiter zeigen zumindest einen Phenylpyridinliganden: Der Phenylpyridinligand ist dabei im Sinne einer Cyclometallisierung an das Zentralatom gebunden, wobei eine direkte Metall-Kohlenstoffbindung ausgebildet wird. Unten sind einige typische Vertreter gezeigt:

Beispiele für cyclometallisierte Iridium basierte Emitter sind:

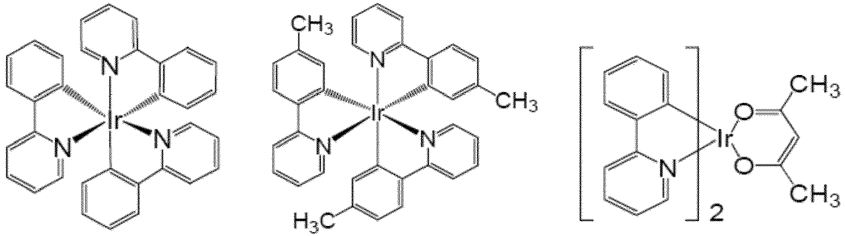

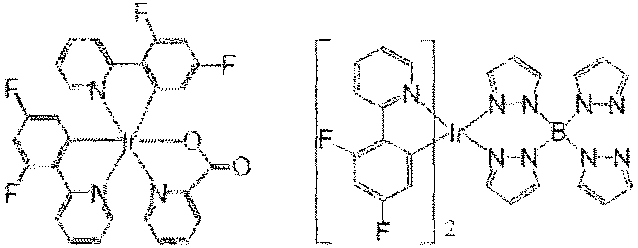

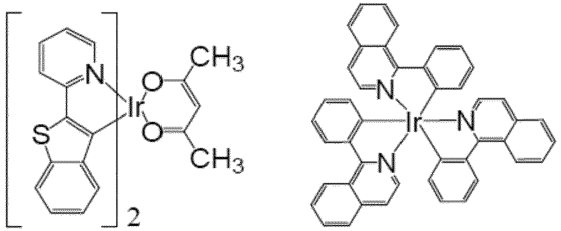

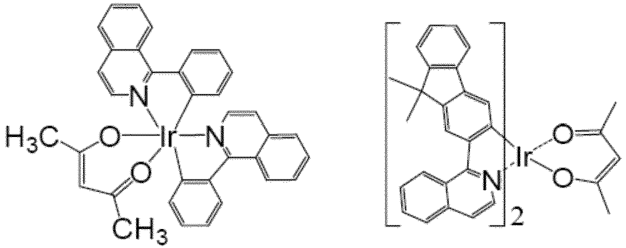

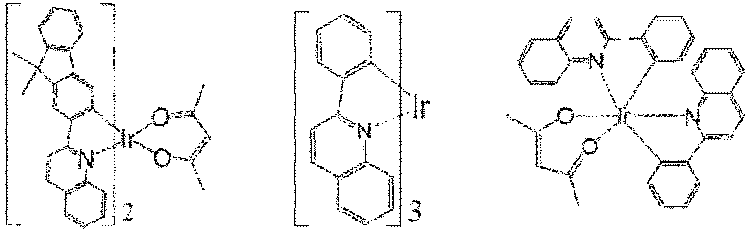

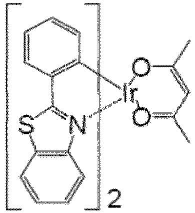

Dies sind typische Beispiele für cyclometallisierte Iridium basierte Emitter. Ein weiterer Vertreter ist der Iridiumcarbenkomplex:

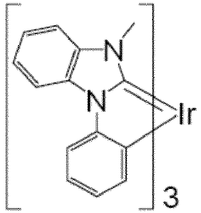

Emittierende Systeme sind nicht auf Iridium beschränkt. Es gibt auch Beispiele für neutrale Emitter mit Lanthaniden, insbesondere Europium und andere Schwermetalle, beispielsweise Osmium.

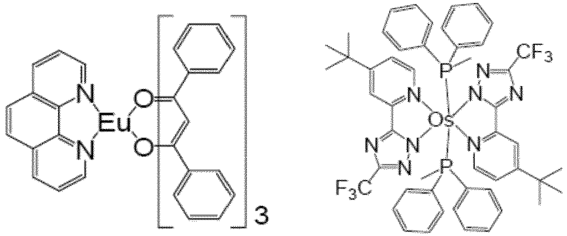

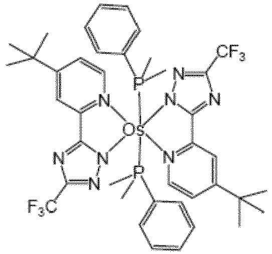

Die gezeigten Grundstrukturen werden können über Guanidinium-Einheiten verbunden werden, so dass ionische Emitter Iridium-basiert oder nicht-Iridium-basiert, entstehen.

In den oben gezeigten Formeln ist der Phenylpyridinligand bevorzugt im Sinne einer Cyclometallisierung an das Zentralatom gebunden, wobei eine direkte Metall-Kohlenstoffbindung ausgebildet wird. Hier sind einige typische Iridiumkomplexe mit Cyclometallisierung gezeigt. Fluorinierung des Phenylpyridin Liganden shiftet die Emission im Spektrum in Richtung blau. Bekannte Beispiele für die fluorinierten Spezies sind die bis(2,4-difluorophenyl-2-pyridyl)-Iridium(III)-picolinate (FIrPic) oder Bis(2,4-difluorophenylpyridinato)tetrakis(1-pyrazolyl)borate iridium III (FIr₆).

Für OLED Anwendungen gibt es sehr viele Emitter, beispielhaft seien hier typische zyklometallisierende Liganden genannt, wie sie beispielsweise in WO2005097942A1, WO2006013738A1, WO2006098120A1, WO2006008976A1, WO2005097943A1, (Konica Minolta) oder, US 6,902,830, US 7,001,536, US 6,830,828 (UDC) beschrieben werden. Sie sind alle über eine N^C- Einheit an Iridium oder ein anderes Zentralatom gebunden. Beispiel: 2-Phenylpyridin oder 2-Phenylimidazol und verwandte Strukturen, wie beispielsweise Benzimidazol oder Phenanthridin. Besonders die 2-Phenylimidazol-Abkömmlinge sind bekannt für eine Verschiebung der Emission in den blaugrünen bis blauen Spektralbereich.

Die bekannten Liganden L können beispielsweise über eine weitere Carbenfunktionalität verfügen, die als Quelle tiefer blauer Emission dient. Beispiele für diese Liganden L sind in den Veröffentlichungen WO200519373, EP1692244B1 oder WO 2008000726A1 zu finden.

Weitere Beispiele möglicher Liganden L sind aus den Veröffentlichungen EP1904508A2, WO2007004113A2, WO2007004113R4A3 bekannt, wobei diese Liganden L auch im Rahmen von geladenen Metallkomplexen, die zumindest einen Phenylpyridin Ligand mit entsprechenden Donorgruppen wie Dimethylamino haben, gezeigt werden. Diese Verbindungen zeigen ein erhöhtes LUMO Niveau des Komplexes, wobei Akzeptorgruppen wie beispielsweise 2,4 Difluoro, in den Phenylring eingeführt werden, um das Niveau des HOMO-Orbitals zu erniedrigen. Es wird gezeigt, dass man durch die Variation der Liganden und deren Substituenten die Emissionsfarbe durch das ganze sichtbare Spektrum hindurch variieren kann.

Aus der noch unveröffentlichten DE 10 2009 031 683.3 sind Iridiumcarbenkomplexe bekannt. Darüber hinaus sind emittierende Systeme natürlich nicht auf Iridium beschränkt, vielmehr können neutrale Emitter auch mit Lanthaniden, insbesondere Europium und andere Schwermetalle wie Osmium, gebildet werden.

Beispiele für bekannte neutrale Emitter:

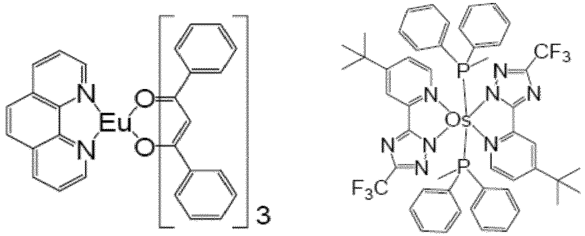

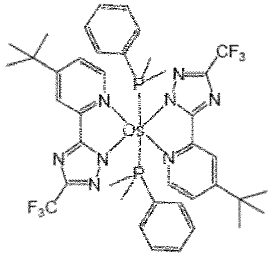

Generell sind alle Reste R = unabhängig voneinander- H, verzweigte Alkylreste, unverzweigte Alkylreste, kondensierte Alkylreste, ringförmige Alkylreste, vollständig oder teilweise substituierte unverzweigte, verzweigte, kondensierte und/oder ringförmige Alkylreste, Alkoxygruppen, Amine, Amide, Ester, Carbonate, Aromaten, vollständig oder teilweise substituierte Aromaten, Heteroaromaten, kondensierte Aromaten, vollständig oder teilweise substituierte kondensierte Aromaten, Heterocyclen, vollständig oder teilweise substituierte Heterocyclen, kondensierte Heterocyclen, Halogene, Pseudohalogene.

Alle Substituenten R₁, R₂, R+ können unabhängig von einander ausgewählt sein aus den oben genannten Resten, es handelt sich bevorzugt um C₁ bis C₂₀, kondensierte z.B. decahydronaphtyl-, adamantyl-, cyclischer, cyclohexyl-, oder voll oder teilweise substituierter Alkylrest, bevorzugt C₁ bis C₂₀. Diese Ketten oder Gruppen können verschiedene Endgruppen tragen, beispielsweise geladene Endgruppen wie SOₓ⁻' NR⁺ und so weiter.

Die Alkylreste können wiederum Gruppen wie Ether, Ethoxy-, Methoxy-, etc. Ester-, Amid-, Carbonat-, etc. oder Halogene, bevorzugt Fluor, tragen. R₁, R₂ und R₃ sollen aber nicht auf Alkylreste beschränkt sein, sondern kann ebenso gut substituierte oder unsubstituierte aromatische Systeme umfassen, wie beispielsweise Phenyl-, Biphenyl-, Naphtyl-, Phenanthryl-, Benzyl-, und so weiter.

Im Folgenden werden Guanidinium-Kationen beschrieben, die Teil eines phosphoreszenten Emittersystems sind.

Dazu wird gemäß der oben gezeigten allgemeinen Formel **I** mindestens einer der Aromaten A mittels eines Spacers an eine Guanidiniumeinheit gekoppelt. Dadurch wird mindestens einer der klassischen Substituenten in den Liganden durch eine Guanidiniumeinheit ersetzt.

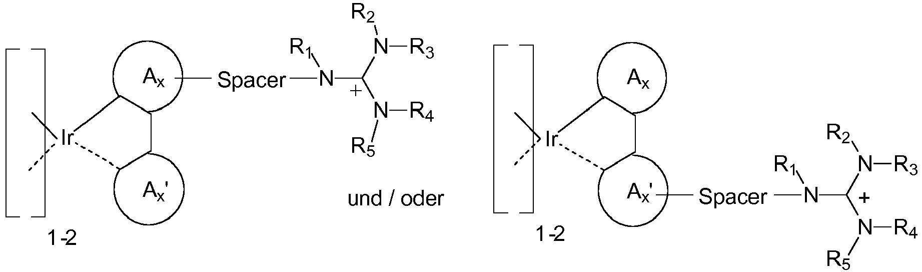

Allgemeine Formel **II** der Emittermaterialien, wobei x = 1 - 3.

Es können nicht erfindungsgemäß andere Metalle wie beispielsweise aber nicht einschränkend Eu, Nd, Gd, Re, Os, Pt, Au, Hg und Ru, Rh, Pd, Ag verwendet werden.

Die beiden weiteren Liganden können, können unabhängig voneinander analog den bekannten Imidazolinium substituierten Liganden ausgebildet sein oder, werden aus den klassischen zyklometallisierenden Liganden ausgewählt, wie sie beispielsweise in WO2005097942A1, WO2006013738A1, WO2006098120A1 WO2006008976A1, WO2005097943A1, (Konica Minolta) oder,
US 6,902,830, US 7,001,536, US 6,830,828 (UDC) beschrieben werden. Sie sind alle über eine N^C- Einheit an Iridium gebunden (Beispiel: 2-Phenylpyridin oder 2-Phenylimidazol und verwandte Strukturen, wie beispielsweise Benzimidazol oder Phenanthridin).

Als Spacer können eingesetzt werden:
a) nicht erfindungsgemäße aliphatische Ketten wie -(CH₂)ₙ-wobei n = 1 -20, bevorzugt n = 1 - 5,
b) nicht erfindungsgemäße fluorierte Alkylketten mit 1 - 12 Kohlenstoffatomen in der Kette, besonders bevorzugt 6 -10,
c) ungesättigte Alkylketten mit 1 - 20 Kohlenstoffatomen und konjugierten Doppelbindungen oder nicht erfindungsgemäße nicht-konjugierten Doppelbindungen,
d) ungesättigte Alkylketten mit 1 - 20 Kohlenstoffatomen und konjugierten Dreifachbindungen oder nicht erfindungsgemäße nicht-konjugierten Dreifachbindungen, auch in Verbindung mit Aromaten,
e) anstelle einer Alkylkette kann auch eine nicht erfindungsgemäße Polyethylenglykol-, Polyethylendiamin-, Polyester-, Polyurethan-, Polyvinylidenphenylenkette Verwendung finden,
f) nicht erfindungsgemäße Ketten, die Aromaten enthalten, insbesondere lässt sich damit auch die Geometrie der Imidazoliniumgruppe im Vergleich zum Emitter einstellen,
g) gemischte Varianten aus a - g,
h) weitere nicht erfindungsgemäße Spacer, die für den Fachmann nahe liegen aber hier nicht extra erwähnt sind.

Die einzige Figur zeigt den Aufbau einer OLEEC schematisch.

Eine OLEEC 7 ist prinzipiell einfacher als die OLED aufgebaut und in den meisten Fällen durch ein einfaches Einbringen einer organischen Schicht 3 zwischen zwei Elektroden 2 und 4 und nachfolgender Verkapselung 5 realisierbar. Beim Anlegen von Spannung tritt Licht 6 aus. Die bevorzugt eine aktive emittierende Schicht 3 einer OLEEC besteht aus einer Matrix, in die eine emittierende Spezies eingebettet ist. Die Matrix kann aus einem Isolator oder aus einem Material bestehen, das entweder ein Ionenleiter mit Elektrolyt-Eigenschaften oder eine inerte Matrix (Isolator) ist. Die emittierende Spezies ist/sind ein oder mehrere ionische Übergangsmetallkomplexe (ionic transistion metal complexes, kurz: iTMC), wie beispielsweise die Verbindungen mit einem Guanidinium-Kation und/oder ist in einem Matrixmateril eingebettet, das gemäß vorliegender Erfindung auch wieder ein Guanidinium-Kation enthalten kann.

Auf dem transparenten Substrat 1 befindet sich die untere Elektrodenschicht 2, beispielsweise die Anode. Darüber befinden sich die eigentlich aktive emittierende Schicht 3 und darüber die zweite Elektrode 4. Zur besseren Performance und Verarbeitung wird das Emittermaterial (iTMC), das die aktive Schicht 3 bildet, sprich die phosphoreszente Metallkomplexverbindung, gemeinsam mit einem Matrixmaterial in einem Lösemittel gelöst. Bevorzugt aber nicht einschränkend werden folgende Lösemittel eingesetzt: Acetonitril, Tetrahydrofuran (THF), Toluol, Ethylenglycoldiethylether, Butoxyethanol, Chlorbenzol, Propylenglycolmethyletheracetat, weitere organische und anorganische sowie polare oder unpolare und Lösungsmittelgemische sind auch im Sinne der Erfindung einsetzbar. Die löslichen Matrixmaterialien, die in Verbindung mit iTMCs eingesetzt werden, sind beispielsweise Polymere, Oligomere und ionische Flüssigkeiten.

Als Matrixmaterialien für den Aufbau einer OLEEC gemäß der Erfindung werden bevorzugt ionische Flüssigkeiten verwendet, die ebenfalls eine Guanidiniumeinheit enthalten. Die erfindungsgemäßen Emitter lösen sich daher sehr gut in der Matrix.

Als Matrixmaterialien für den Aufbau einer OLEEC werden ionische Flüssigkeiten verwendet, die ebenfalls bevorzugt aber nicht einschränkend eine Guanidiniumeinheit enthalten. Alternativ und ergänzend können Matrixmaterialien Verwendung finden, die beispielsweise Imidazoliniumkationen enthalten. Die erfindungsgemäßen Emitter lösen sich daher sehr gut in der Matrix.

Bevorzugt aber nicht einschränkend können die "einfachen" Anionen ausgewählt werden aus: Fluorid, Chlorid, Bromid, Jodid, Sulfat, Phosphat, Carbonat, Trifluormethansulfonat, Trifluoracetat, Tosylat, Bis(trifluormethylsulfon)imid, Tetraphenylborat, B₉C₂H₁₁²; Hexafluorophosphat, Tetrafluoroborat, Hexafluoroantimonat, Tetrapyrazolatoborat. Im Sinne der Erfindung sind auch komplexe Anionen wie beispielsweise Fe(CN₆³⁻, Fe(CN)₆4-, Cr(C₂O₄)³⁻, Cu(CN)₄³⁻, Ni(CN)4²⁻.

Möglich ist auch eine Kopplung der Guanidiniumeinheit mit Sulfonat, Pyridinium, Imidazolinium.

Tae-Hyuk Kwon, Yong Ho Oh, Ik-Soo Shin, and Jon-In Hong, Adv. Mater. 19,1-7, 2009

P. Coppo, M. Duati, V.N. Kozhevnikov, J. W. Hofstraat, L. De Cola, Angew. Chemie Int. Engl. 44, 1806, 2005

In der nachfolgenden Tabelle sind einige typische Vertreter ionischer Flüssigkeiten aufgelistet:
1-Benzyl-3-methylimidazolium hexafluorophosphate
1-Butyl-2,3-dimethylimidazolium hexafluorophosphate
1-Butyl-3-methylimidazolium hexafluorophosphate
1-Ethyl-3-methylimidazolium hexafluorophosphate
1-Hexyl-3-methylimidazolium hexafluorophosphate
1-Butyl-1-(3,3,4,4,5,5,6,6,7,7,8,8,8-tridecafluorooctyl)imidazolium hexafluorophosphate
1-Methyl-3-(3,3,4,4,5,5,6,6,7,7,8,8,8-tridecafluorooctyl)imidazolium hexafluorophosphate
1-Methyl-3-octylimidazolium hexafluorophosphate
1-Butyl-2,3-dimethylimidazolium tetrafluoroborate
1-Butyl-3-methylimidazolium tetrafluoroborate
1-Ethyl-3-methylimidazolium tetrafluoroborate
1-Hexyl-3-methylimidazolium tetrafluoroborate
1-Methyl-3-octylimidazolium tetrafluoroborate
1-Butyl-3-methylimidazolium trifluoromethanesulfonate
1-Ethyl-3-methylimidazolium trifluoromethanesulfonate
1,2,3-Trimethylimidazolium trifluoromethanesulfonate
1-Ethyl-3-methyl-imidazolium bis(pentafluoroethylsulfonyl)imide
1-Butyl-3-methylimidazolium bis(trifluoromethylsulfonyl)imide
1-Butyl-3-methylimidazolium methanesulfonate Tetrabutylammonium bis-trifluoromethanesulfonimidate Tetrabutylammonium methanesulfonate Tetrabutylammonium nonafluorobutanesulfonate Tetrabutylammonium heptadecafluorooctanesulfonate Tetrahexylammonium tetrafluoroborate Tetrabutylammonium trifluoromethanesulfonate Tetrabutylammonium benzoate Tetrabutylammonium chloride Tetrabutylammonium bromide
1-Benzyl-3-methylimidazolium tetrafluoroborate Trihexyltetradecylphosphonium hexafluorophosphate Tetrabutylphosphonium methanesulfonate Tetrabutylphosphonium tetrafluoroborate Tetrabutylphosphonium bromide
1-Butyl-3-methylpyridinium bis(trifluormethylsulfonyl)imide
1-Butyl-4-methylpyridinium hexafluorophosphate
1-Butyl-4-methylpyridinium tetrafluoroborate Sodium tetraphenylborate Tetrabutylammonium tetraphenylborate Sodium tetrakis(1-imidazolyl)borate Cesium tetraphenylborate

Mit dieser Erfindung werden Matrixmaterialien und Emitter für Anwendungen in organischen elektrochemischen lichtemittierenden Zellen beschrieben, die eine Guanidinium-Kationeinheit enthalten. Guanidiniumgruppen als kationisches System versprechen ein breites elektrochemisches Fenster und damit stabile Bauelemente. Der Zugang zu Guanidiniumsystemen ist in der Literatur detailliert beschrieben.

## Patentansprüche

1. Verwendung eines Salzes, das ein Guanidinium-Kation enthält, als phosphoreszentes Emittersystem in einem lichtemittierenden organischen elektronischen Bauelement, wobei das lichtemittierende organische elektronische Bauelement eine organische elektrochemische Zelle ist, wobei das Guanidinium-Kation enthaltende Salz die folgende Struktureinheit zeigt: 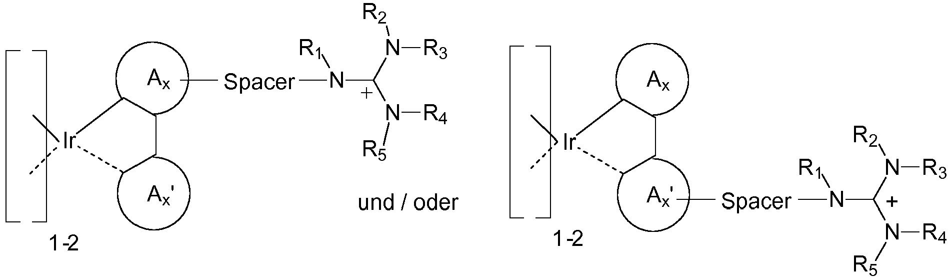 wobei
A = ein substituierter oder unsubstituierter Aromat oder Heteroaromat ist, der zu den genannten Bindungsverhältnissen befähigt ist,
als Spacer eingesetzt werden:
a) ungesättigte Alkylketten mit 1 - 20 Kohlenstoffatomen und konjugierten Doppelbindungen,
b) ungesättigte Alkylketten mit 1 - 20 Kohlenstoffatomen und konjugierten Dreifachbindungen, oder
c) gemischte Varianten aus a und b,
wobei die Substituenten R₁ bis R₅ unabhängig voneinander aliphatisch, aromatisch oder komplex aufgebaut sein können und insbesondere
R = unabhängig voneinander H, gesättigte oder ungesättigte, verzweigte Alkylreste, unverzweigte Alkylreste, kondensierte Alkylreste, ringförmige Alkylreste, vollständig oder teilweise substituierte unverzweigte, verzweigte, kondensierte und/oder ringförmige Alkylreste, Alkoxygruppen, Amine, Amide, Ester, Carbonate, Aromaten, vollständig oder teilweise substituierte Aromaten, Heteroaromaten, kondensierte Aromaten, vollständig oder teilweise substituierte kondensierte Aromaten, Heterocyclen, vollständig oder teilweise substituierte Heterocyclen, kondensierte Heterocyclen, Halogene, Pseudohalogene sein kann.

2. Verwendung nach dem vorstehenden Anspruch, wobei das das Guanidinium-Kation enthaltende Salz in der aktiven organischen Schicht der organischen elektrochemischen Zelle enthalten ist.

3. Verwendung nach einem der vorstehenden Ansprüche, wobei das das Guanidinium-Kation enthaltende Salz in dem Emittermaterial und in dem Matrixmaterial der aktiven organischen Schicht des lichtemittierenden Bauelements enthalten ist.

4. Verwendung nach einem der vorstehenden Ansprüche, wobei das das das Guanidinium-Kation enthaltende Salz die folgende Struktureinheit zeigt: 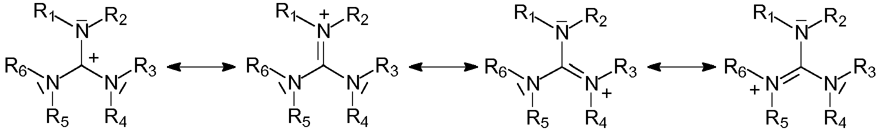 wobei die Substituenten R₁ bis R₆ unabhängig voneinander aliphatisch, aromatisch oder komplex aufgebaut sein können und insbesondere
R = unabhängig voneinander- H, gesättigte oder ungesättigte, verzweigte Alkylreste, unverzweigte Alkylreste, kondensierte Alkylreste, ringförmige Alkylreste, vollständig oder teilweise substituierte unverzweigte, verzweigte, kondensierte und/oder ringförmige Alkylreste, Alkoxygruppen, Amine, Amide, Ester, Carbonate, Aromaten, vollständig oder teilweise substituierte Aromaten, Heteroaromaten, kondensierte Aromaten, vollständig oder teilweise substituierte kondensierte Aromaten, Heterocyclen, vollständig oder teilweise substituierte Heterocyclen, kondensierte Heterocyclen, Halogene, Pseudohalogene sein kann.

5. Organische elektrochemische Zelle, zumindest zwei Elektrodenschichten und dazwischen eine aktive organische Schicht aufweisend, wobei in der aktiven organischen Schicht ein Guanidinium-Kation enthaltendes Salz als phosphoreszentes Emittersystem vorhanden ist,
wobei das das Guanidinium-Kation enthaltende Salz die folgende Struktureinheit zeigt: 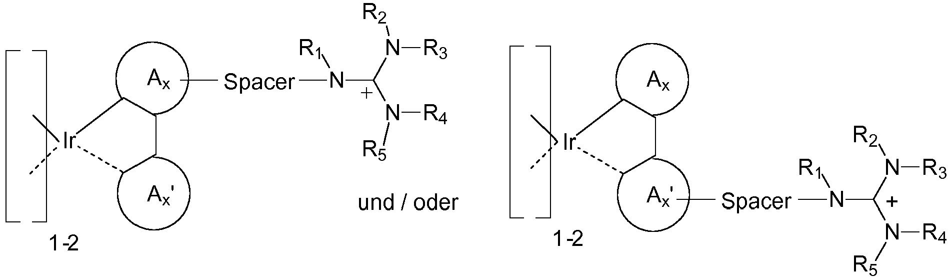 wobei
A = ein substituierter oder unsubstituierter Aromat oder Heteroaromat ist, der zu den genannten Bindungsverhältnissen befähigt ist,
als Spacer eingesetzt werden:
a) ungesättigte Alkylketten mit 1 - 20 Kohlenstoffatomen und konjugierten Doppelbindungen,
b) ungesättigte Alkylketten mit 1 - 20 Kohlenstoffatomen und konjugierten Dreifachbindungen, oder
c) gemischte Varianten aus a und b,
wobei die Substituenten R₁ bis R₅ unabhängig voneinander aliphatisch, aromatisch oder komplex aufgebaut sein können und insbesondere
R = unabhängig voneinander- H, gesättigte oder ungesättigte, verzweigte Alkylreste, unverzweigte Alkylreste, kondensierte Alkylreste, ringförmige Alkylreste, vollständig oder teilweise substituierte unverzweigte, verzweigte, kondensierte und/oder ringförmige Alkylreste, Alkoxygruppen, Amine, Amide, Ester, Carbonate, Aromaten, vollständig oder teilweise substituierte Aromaten, Heteroaromaten, kondensierte Aromaten, vollständig oder teilweise substituierte kondensierte Aromaten, Heterocyclen, vollständig oder teilweise substituierte Heterocyclen, kondensierte Heterocyclen, Halogene, Pseudohalogene sein kann.

## Claims

1. Use of a salt containing a guanidinium cation as phosphorescent emitter system in a light-emitting organic electronic component, wherein the light-emitting organic electronic component is an organic electrochemical cell, wherein the guanidinium cation-containing salt exhibits the following structural unit: 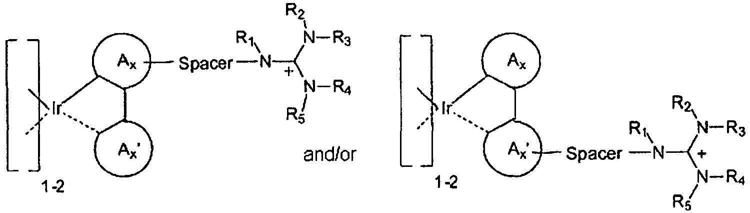 where
A = a substituted or unsubstituted aromatic or heteroaromatic capable of the bonding conditions specified,
the spacers used are:
a) unsaturated alkyl chains having 1-20 carbon atoms and conjugated double bonds,
b) unsaturated alkyl chains having 1-20 carbon atoms and conjugated triple bonds, or
c) mixed variants of a and b,
where the substituents R₁ to R₅ may each independently be of aliphatic, aromatic or complex structure and may especially be
R = independently H, saturated or unsaturated, branched alkyl radicals, unbranched alkyl radicals, fused alkyl radicals, cyclic alkyl radicals, completely or partially substituted unbranched, branched, fused and/or cyclic alkyl radicals, alkoxy groups, amines, amides, esters, carbonates, aromatics, completely or partially substituted aromatics, heteroaromatics, fused aromatics, completely or partially substituted fused aromatics, heterocycles, completely or partially substituted heterocycles, fused heterocycles, halogens, pseudohalogens.

2. Use according to the preceding claim, wherein the salt containing the guanidinium cation is present in the active organic layer of the organic electrochemical cell.

3. Use according to either of the preceding claims, wherein the salt containing the guanidinium cation is present in the emitter material and in the matrix material of the active organic layer of the light-emitting component.

4. Use according to any of the preceding claims, wherein the salt containing the guanidinium cation exhibits the following structural unit: 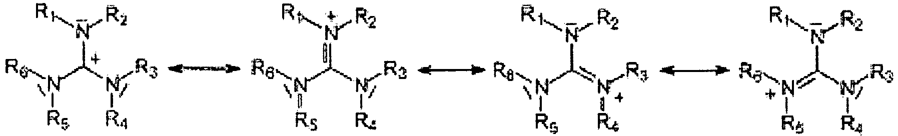
where the substituents R₁ to R₆ may each independently be of aliphatic, aromatic or complex structure and may especially be
R = independently H, saturated or unsaturated, branched alkyl radicals, unbranched alkyl radicals, fused alkyl radicals, cyclic alkyl radicals, completely or partially substituted unbranched, branched, fused and/or cyclic alkyl radicals, alkoxy groups, amines, amides, esters, carbonates, aromatics, completely or partially substituted aromatics, heteroaromatics, fused aromatics, completely or partially substituted fused aromatics, heterocycles, completely or partially substituted heterocycles, fused heterocycles, halogens, pseudohalogens.

5. Organic electrochemical cell having at least two electrode layers and an active organic layer in between, wherein a guanidinium cation-containing salt is present as phosphorescent emitter system in the active organic layer,
wherein the salt containing the guanidinium cation exhibits the following structural unit: 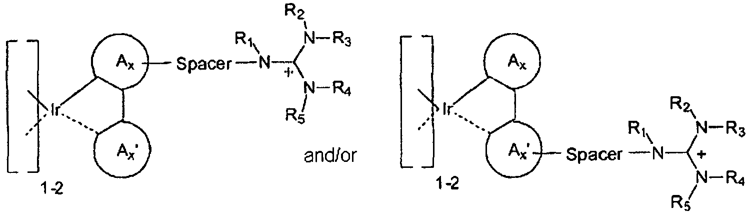 where
A = a substituted or unsubstituted aromatic or heteroaromatic capable of the bonding conditions specified,
the spacers used are:
a) unsaturated alkyl chains having 1-20 carbon atoms and conjugated double bonds,
b) unsaturated alkyl chains having 1-20 carbon atoms and conjugated triple bonds, or
c) mixed variants of a and b,
where the substituents R₁ to R₅ may each independently be of aliphatic, aromatic or complex structure and may especially be
R = independently H, saturated or unsaturated, branched alkyl radicals, unbranched alkyl radicals, fused alkyl radicals, cyclic alkyl radicals, completely or partially substituted unbranched, branched, fused and/or cyclic alkyl radicals, alkoxy groups, amines, amides, esters, carbonates, aromatics, completely or partially substituted aromatics, heteroaromatics, fused aromatics, completely or partially substituted fused aromatics, heterocycles, completely or partially substituted heterocycles, fused heterocycles, halogens, pseudohalogens.

## Revendications

1. Utilisation d'un sel, qui contient un cation guanidinium, en tant que système émetteur phosphorescent dans un composant électronique organique émetteur de lumière, le composant électronique organique émetteur de lumière étant une cellule électrochimique organique, le sel contenant un cation de guanidinium présentant l'unité structurale suivante : 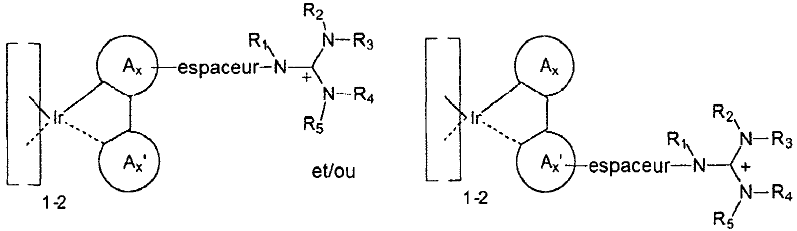 dans laquelle
A = un groupe aromatique ou hétéroaromatique substitué ou non substitué, qui est apte aux liaisons indiquées,
en tant qu'espaceurs, sont utilisés :
a) des chaînes alkyle insaturées contenant 1 à 20 atomes de carbone et des doubles liaisons conjuguées,
b) des chaînes alkyle insaturées contenant 1 à 20 atomes de carbone et des triples liaisons conjuguées, ou
c) des variantes mélangées de a et b,
les substituants R₁ à R₅ pouvant indépendamment les uns des autres être de structure aliphatique, aromatique ou complexe, et notamment
les R pouvant représenter indépendamment les uns des autres H, des radicaux alkyle ramifiés, saturés ou insaturés, des radicaux alkyle non ramifiés, des radicaux alkyle condensés, des radicaux alkyle cycliques, des radicaux alkyle non ramifiés, ramifiés, condensés et/ou cycliques substitués en totalité ou en partie, des groupes alcoxy, des amines, des amides, des esters, des carbonates, des groupes aromatiques, des groupes aromatiques substitués en totalité ou en partie, des groupes hétéroaromatiques, des groupes aromatiques condensés, des groupes aromatiques condensés substitués en totalité ou en partie, des hétérocycles, des hétérocycles substitués en totalité ou en partie, des hétérocycles condensés, des halogènes, des pseudohalogènes.

2. Utilisation selon la revendication précédente, dans laquelle le sel contenant le cation guanidinium est contenu dans la couche organique active de la cellule électrochimique organique.

3. Utilisation selon l'une quelconque des revendications précédentes, dans laquelle le sel contenant le cation guanidinium est contenu dans le matériau émetteur et dans le matériau de matrice de la couche organique active du composant émetteur de lumière.

4. Utilisation selon l'une quelconque des revendications précédentes, dans laquelle le sel contenant le cation guanidinium présente l'unité structurale suivante : 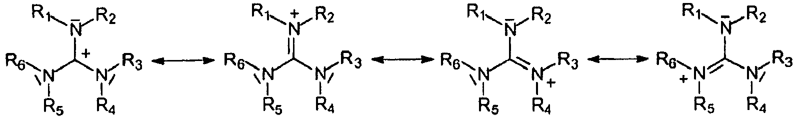 dans laquelle les substituants R₁ à R₆ peuvent indépendamment les uns des autres être de structure aliphatique, aromatique ou complexe, et notamment les R peuvent représenter indépendamment les uns des autres H, des radicaux alkyle ramifiés, saturés ou insaturés, des radicaux alkyle non ramifiés, des radicaux alkyle condensés, des radicaux alkyle cycliques, des radicaux alkyle non ramifiés, ramifiés, condensés et/ou cycliques substitués en totalité ou en partie, des groupes alcoxy, des amines, des amides, des esters, des carbonates, des groupes aromatiques, des groupes aromatiques substitués en totalité ou en partie, des groupes hétéroaromatiques, des groupes aromatiques condensés, des groupes aromatiques condensés substitués en totalité ou en partie, des hétérocycles, des hétérocycles substitués en totalité ou en partie, hétérocycles condensés, des halogènes, des pseudohalogènes.

5. Cellule électrochimique organique, comprenant au moins deux couches d'électrode et entre elles une couche organique active, un sel contenant un cation guanidinium étant présent dans la couche organique active en tant que système émetteur phosphorescent,
le sel contenant le cation guanidinium présentant l'unité structurale suivante : 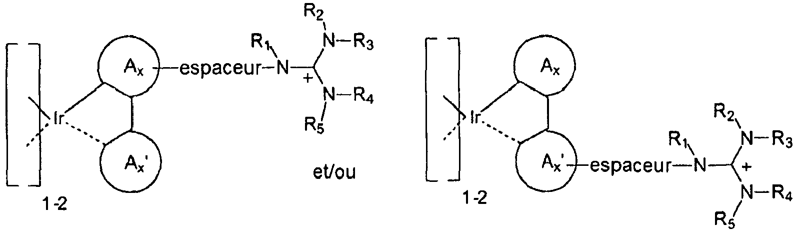 dans laquelle
A = un groupe aromatique ou hétéroaromatique substitué ou non substitué, qui est apte aux liaisons indiquées,
en tant qu'espaceurs, sont utilisés :
a) des chaînes alkyle insaturées contenant 1 à 20 atomes de carbone et des doubles liaisons conjuguées,
b) des chaînes alkyle insaturées contenant 1 à 20 atomes de carbone et des triples liaisons conjuguées, ou
c) des variantes mélangées de a et b,
les substituants R₁ à R₅ pouvant indépendamment les uns des autres être de structure aliphatique, aromatique ou complexe, et notamment
les R pouvant représenter indépendamment les uns des autres H, des radicaux alkyle ramifiés, saturés ou insaturés, des radicaux alkyle non ramifiés, des radicaux alkyle condensés, des radicaux alkyle cycliques, des radicaux alkyle non ramifiés, ramifiés, condensés et/ou cycliques substitués en totalité ou en partie, des groupes alcoxy, des amines, des amides, des esters, des carbonates, des groupes aromatiques, des groupes aromatiques substitués en totalité ou en partie, des groupes hétéroaromatiques, des groupes aromatiques condensés, des groupes aromatiques condensés substitués en totalité ou en partie, des hétérocycles, des hétérocycles substitués en totalité ou en partie, des hétérocycles condensés, des halogènes, des pseudohalogènes.
